# EUROPEAN PATENT SPECIFICATION

(11) **EP 0 652 857 B1**
(45) Date of publication and mention of the grant of the patent: **15.10.1997**
(21) Application number: 93918272.1
(22) Date of filing: 23.07.1993
(51) Int. Cl.: C07C 17/358, C07C 19/12

(54) **PROCESS FOR ISOMERIZING 1,1,2-TRICHLOROTRIFLUOROETHANE**
VERFAHREN ZUR ISOMERISIERUNG VON 1,1,2-TRICHLORTRIFLUORETHAN
PROCEDE D'ISOMERISATION DE 1,1,2-TRICHLOROTRIFLUORETHANE

(30) Priority: 31.07.1992 US 923127
(43) Date of publication of application: 17.05.1995
(73) Proprietor: E.I. DU PONT DE NEMOURS AND COMPANY, Wilmington Delaware 19898 (US)
(72) Inventor: GUMPRECHT, William, Henry, Wilmington, DE 19808 (US); HUEBNER, William, Joel, Elkton, MD 21921-4523 (US); NAPPA, Mario, Joseph, Newark, DE 19711-3435 (US)
(74) Representative: Jones, Alan John
(86) International application number: US9306824
(87) International publication number: WO9403417

(56) References cited:
- DE-A- 1 668 346
- US-A- 4 925 993

## Description

This invention relates to processes using activated aluminum trichloride catalysts and more particularly to chlorofluorocarbon isomerizations in the presence of such catalysts.

### BACKGROUND OF THE INVENTION

Chlorofluorocarbons have been widely used as refrigerants and aerosols. Concerns have developed over the release of various chlorofluorocarbons into the atmosphere. Efforts have already begun to eliminate any widespread emission of CCl₂F₂ (CFC-12) and CCl₃F (CFC-11) due to their ability to reach the stratosphere intact. In recent years, increased attention has focused on use of perfluorocarbons, hydrofluorocarbons and hydrochlorofluorocarbons which are considered to have significantly lower potential for contributing to ozone depletion. Of particular interest to the refrigerant industry is the hydrofluorocarbon 1,1,1,2-tetrafluoroethane (HFC-134a). HFC-134a can be derived from the intermediates 1,1,1-trichlorotrifluoroethane (CFC-113a) and 1,1,1,2-tetra-fluorodichloroethane (CFC-114a) These intermediates are, in turn, the isomers of the readily available and chemically more symmetric chlorofluoroethanes, 1,1,2-trichlorotrifluoroethane (CFC-113) and 1,1,2,2-tetra-fluorodichloroethane (CFC-114), respectively.

The isomerization of these more symmetric chlorofluoroethanes by treatment with aluminum trichloride is well documented in the art. For example, U.S. Pat. No. 4,925,993 discloses a process for isomerizing CFC-112 (i.e., CCl₂FCCl₂F) to CFC-112a (i.e., CCl₃CClF₂) or CFC-113 to CFC-113a by contacting the CFC-112 or CFC-113 with an activated aluminum trichloride catalyst. The catalyst is prepared by contacting the chlorofluorocarbon with anhydrous aluminum trichloride in the presence of selected metals. Reportedly, the amount of non-isomerized chlorofluoroethane in the product mixture can be held to less than 450 ppm, and the catalyst can be used for eighteen cycles.

High purity CFC-113a is an advantageous starting material for the preparation of high purity 1,1,1,2-tetrafluoroethane (HFC-134a). More particularly, CFC-113a may be reacted with HF to afford CFC-114a which in turn may be hydrodechlorinated to HFC-134a. Unless an isomer separation process is employed, the presence of substantial amounts CFC-113 in the starting material would eventually lead to the production of a HFC-134a product containing significant amounts of 1,1,2,2-tetrafluoroethane (HFC-134). It has been found that for many applications, the presence of significant amounts of HFC-134 in an HFC-134a product can alter the physical and chemical properties of the product. Thus, there is interest in developing more efficient isomerization methods for obtaining CFC-113a products with negligible amounts (i.e., less than about 500 ppm) of non-isomerized starting material.

### SUMMARY OF THE INVENTION

The invention provides an isomerization process for producing a desirable chlorofluoroethane intermediate of 1,1,1,2-tetrafluoroethane. The process comprises (1) activating a micropulverized anhydrous aluminum trichloride which has a surface area of greater than 0.8 m²/g by treating it with at least 10 g per g of aluminum trichloride of CCl₂FCClF₂ with agitation; and (2) isomerizing an additional amount of CCl₂FCClF₂ by (i) mixing said additional amount of CCl₂FCClF₂ with said activated catalyst with agitation to provide an initial degree of isomerization, and (ii) cooling the mixture of chlorofluorocarbons and catalyst to a temperature of about 20°C with agitation, to provide a product containing CCl₃CF₃. The amount of non-isomerized CCl₂FCClF₂ in the product is generally less than about 400 ppm.

### BRIEF DESCRIPTION OF THE DRAWINGS

Figure 1 is a plot of non-isomerized CFC-113 in CFC-113a product as a function of isomerization cycle for catalyst prepared from AlCl₃ having a surface area of about 0.27 m²/g.

Figure 2 is a plot of non-isomerized CFC-113 in CFC-113a product as a function of isomerization cycle for catalyst prepared from AlCl₃ having a surface area of about 0.39 m²/g.

Figure 3 is a plot of non-isomerized CFC-113 in CFC-113a product as a function of isomerization cycle for catalyst prepared from AlCl₃ having a surface area of about 0.85 m²/g.

Figure 4 is a plot of non-isomerized CFC-113 in CFC-113a product as a function of isomerization cycle for catalyst prepared from micropulverized AlCl₃ having a surface area of about 0.95 m²/g.

### DETAILED DESCRIPTION

This invention provides catalytic isomerization of CCl₂FCClF₂ starting materials. The process uses advantageous activated AlCl₃ catalysts. The isomerization catalyst may be activated in situ (i.e., in the same reactor used for isomerization) by treating micropulverized anhydrous aluminum trichloride with CCl₂FCClF₂. Agitation (e.g., stirring) should be provided during treatment. Micropulverization involves mechanical comminution and may be practiced using a conventional operation such as crushing, ball milling, rod milling or grinding. Typically, the micropulverized anhydrous aluminum trichloride has the following properties: a particle size such that greater than 80% of the material passes through a 100 mesh screen (sieve opening of 0.149 mm); less than 30 ppm iron; less than 0.3% non-volatile material; less than 25 ppm free aluminum; less than 200 ppm water insolubles; and an aluminum chloride purity of greater than 99.6%. In any case, after micropulverization the AlCl₃ should have a surface area greater than 0.8 m²/g (as measured by the well known single-point BET method using nitrogen, for example, a gas mixture of 30% nitrogen and 70% helium). Preferably, the AlCl₃ is sufficiently micropulverized to provide a first order rate constant, after activation, of at least 0.10 minute⁻¹ at boiling under reflux conditions. The weight ratio of chlorofluorocarbon used during activation to micropulverized anhydrous aluminum trichloride is typically from about 10:1 to about 300:1, is preferably from about 20:1 to 70:1, and is most preferably about 50:1.

The aluminum trichloride is preferably activated at a temperature within the range of from 47°C to 52°C when CFC-113 is used. For example, a mixture of micropulverized AlCl₃ and CCl₂FCClF₂ may be heated to boil under reflux (about 48°C) for four hours with stirring. Overheating due to rapid reaction of CFC-113 can be controlled during activation by using CFC-113a (e.g., a ratio of CFC-113a to CFC-113 of about 1.5:1) as a heat sink and diluent to limit the reaction rate. The mixture may then be cooled with stirring to 20°C, at which point the stirring is stopped. After the catalyst has settled to the bottom of the reaction vessel, the supernatant liquid may be decanted from the reaction vessel. A small amount of chlorofluorocarbons (typically between about 10 to about 20 weight percent) is generally retained as a slurry with the activated catalyst.

The activated aluminum trihalide derivative is contacted with additional CCl₂FCClF₂ for isomerization. Typically, stirring is employed during catalyst contact. A catalyst slurry containing the activated catalyst may be transferred to a new reaction vessel for the isomerization, or the additional CCl₂FCClF₂ to be isomerized may be added directly to the vessel containing the freshly activated catalyst.

The reaction vessel(s) used for catalyst activation and isomerization may be any chemically resistant material such as glass (e.g., glass-lined reactors) or carbon steel. Reactor materials which leach contaminants such as undesirable metals into the reaction mixture are preferably avoided.

To achieve a desirable reaction rate, the isomerization is initially conducted with stirring at a temperature of at least 47°C. Preferably, the initial temperature is from 47°C to 52°C (i.e., at or near the boiling temperature for CCl₂FCClF₂). After achieving an initial degree of isomerization, the reaction mixture is cooled to about 20°C, and the isomerization is terminated when the amount of CCl₂FCClF₂ in the reaction vessel is less than 400 ppm, preferably, especially for commercial production, about 350 ppm or less (e.g., about 320 ppm). The catalyst activity, the reaction temperature and the catalyst concentration are preferably controlled so that the desired degree of isomerization is acheived in from about 0.25 to about 4 hours, most preferably about 0.5 hours.

After isomerization, the stirring is stopped, and the catalyst is separated from the product mixture (e.g., by allowing the catalyst to settle to the bottom of the reactor and drawing off or decanting the liquid product). Separation of the isomerization products from other compounds in the organic phase (e.g., disproportionation products and other reaction byproducts) then proceeds by techniques well known to those skilled in the art, such as distillation. Usually 10 to 20 weight percent of the product mixture is retained as a slurry with the catalyst.

The catalyst may be reused until its activity becomes undesirably low. The catalyst may be reused by adding to the slurried catalyst an amount of fresh chlorofluorocarbon approximately equal in weight to the product mixture removed. The weight ratio of chlorofluorocarbon to the original aluminum trichloride catalyst used in each batch is typically between about 30:1 to about 70:1. Separation of the products from the organic phase can be done collectively using a pool of organic phases recovered from a series of isomerizations, or singularly upon the removal of product mixture from the settled catalyst.

Catalyst life can be as important commercially as the rate of the reaction. Surprisingly, the lifetime of catalysts, as indicated by the number of batch runs that a catalyst can be used to produce product within specification (e.g., less than about 400 ppm CFC-113 at 20°C), increases with the surface area of the micropulverized catalyst precursor.

Typically, the catalyst of this invention can be used to isomerize at least 500 kg of CFC-113 per kg of AlCl₃. The isomerization rate prior to cooling (e.g., prior to cooling from an initial isomerization temperature of 47°C or more) is typically at least about 10 kg of CFC-113 per kg of AlCl₃ per hour. When used during isomerization in a weight ratio up to 50 g CFC-113 per g AlCl₃ (e.g., 20 to 40 g CFC-113/g AlCl₃) the catalyst is preferably used for at least 30 isomerization cycles (i.e., isomerization of 30 batches of fresh CCl₂FCClF₂); most preferably, especially for commercial production, at least about 70 isomerization cycles (e.g., about 140 cycles, or even more). Preferably, the catalyst is used to isomerize at least 1000 kg CCl₂FCClF₂ per kg of AlCl₃, most preferably, especially for commercial production, at least about 4000 kg CCl₂FCClF₂ per kg AlCl₃ (e.g., about 5000 kg CCl₂FCClF₂ per kg AlCl₃, or more). Preferably, the isomerization rate of CCl₂FCClF₂ prior to cooling is at least about 25 kg of said chlorofluorocarbon per kg AlCl₃ per hour.

The isomerization normally proceeds at atmospheric pressure. However, since the autocatalytic reaction between anhydrous aluminum trichloride and certain chlorofluorocarbons, such as CCl₂FCClF₂, can be highly exothermic and can present thermal control problems, especially at high temperatures under adiabatic conditions, the process may be performed in a suitable pressure vessel. Adequate heat removal to prevent overheating of the catalyst is desirable to achieve the most active, long-lived catalyst.

The product produced by isomerization of CCl₂FCClF₂ starting material is a high purity composition of CCl₃CF₃ (i.e., the product produced consists essentially of CCl₃CF₃ containing less than about 400 ppm CCl₂FCClF₂).

Practice of the invention is further illustrated by the following non-limiting examples.

### EXAMPLE 1

### Reaction Rate Study

### CCl₂FCClF₂ (CFC-113) Isomerization

### A. Catalyst Activation

A 250 mL Erlenmeyer flask was equipped with a hot plate/stirrer, Teflon® (polytetrafluoroethylene) stirring bar, Teflon® sleeves, an inlet port capped with a Teflon®-coated septum, a condenser with a nitrogen inlet tube and a 250 mL addition funnel fitted with a Teflon® stopcock and connected to a Krytox® (perfluorinated polyether) oil bubbler. All the glassware was oven-dried prior to use. The Erlenmeyer flask was flushed with dry nitrogen and capped with a glass stopper. The flask was charged with AlCl₃ (1.0 g) in a nitrogen-blanketed dry box. The flask was capped with a glass stopper, removed from the dry box and quickly attached to the reflux condenser purged with N₂, and the addition funnel. A thin wire (1/32", 0.79 mm) thermocouple was inserted through the septum so that it was located in the vapor space of the reaction (in some experiments, a thermocouple was inserted in a thermowell). A continuous and generous N₂ purge was maintained through the nitrogen inlet. Water maintained at 10°C in a low temperature bath was circulated through the condenser. The addition funnel was charged with 270 g (about 170 mL) of high-purity CFC-113. The stirring rate was set at 500 rpm, and the N₂ purge reduced to a slow bubbling rate exiting the system. After the addition (10 20 minutes), the heating was set at 85°C, and the time and temperature noted. The mixture was heated to boil under reflux (time is variable depending on the activity of the catalyst). The vapor was sampled through the septum when the reflux was a steady stream coming off the condenser tip. This was noted as the starting point of the activation. The reaction was sampled at appropriate intervals to yield a conversion curve; for each point the time was recorded to the nearest tenth of a minute. The reaction was shut down when the reaction composition did not change significantly as determined by vapor-phase GC analyses.

### B. Isomerisation Procedure

Most of the liquid was removed from the reactor with an 18 gauge needle and a 50 mL syringe, an additional change of 270 g CFC-113 was added to the reactor, and the above process was repeated.

### C. Reaction Rate

The activation and isomerization reactions described above were carried out using a series of AlCl₃ samples including both standard, commercially available AlCl₃ and micropulverized AlCl₃. All the micropulverized AlCl₃ samples possessed a particle size such that 80% of the material passed through 100 mesh screen (sieve opening of 0.149 mm). Surface areas for each of the AlCl₃ samples were measured using the standard BET nitrogen sorption method by degassing about one gram (g) of the sample at room temperature using helium flow in a Micromeritics Desorb 2300B system, measuring adsorption and desorption on a Micromeritics Flowsorb II 2300 system with a mixed gas of 30% nitrogen and 70% helium, and calculating the surface area.

The first-order rate constant for the conversion of CCl₂FCClF₂ (CFC-113) to CCl₃CF₃ (CFC-113a) was measured for the first isomerization for each sample, and the results are shown in Table 1. The data show that the races of isomerization are faster for catalyst precursors with higher surface areas. Moreover, the micropulverized AlCl₃, always performed better (i.e., faster) than standard AlCl₃.

**TABLE 1**

| Surface Area (m²/g) prior to activation | k_{obs} (min⁻¹) | AlCl₃ |
|---|---|---|
| 0.24 | 0.028 | Standard |
| 0.26 | 0.033 | Standard |
| 0.32 | 0.039 | Standard |
| 0.35 | 0.027 | Standard |
| 0.39 | 0.048 | Standard |
| 0.66 | 0.038 | Standard |
| 0.68 | 0.092 | Standard |
| 0.83 | 0.127 | Micropulverized |
| 0.91 | 0.114 | Micropulverized |
| 0.98 | 0.112 | Micropulverized |
| 1.05 | 0.153 | Micropulverized |
| 1.06 | 0.17 | Micropulverized |
| 1.08 | 0.139 | Micropulverised |

### EXAMPLE 2

### Catalyst Life Study

### CCl₂FCClF₂ (CFC-113) Isomerization

### A. Catalyst Activation

The modified Erlenmeyer flask described above was flushed (septum attached) with dry nitrogen. While flushing, it was quickly stoppered and placed in a dry box with a glass vial, a wide-bore glass funnel and a scoupula. A small amount of AlCl₃ was placed in the vial to coat the internal walls. Some AlCl₃ was also used to coat the walls of the funnel. AlCl₃ was weighed in the coated glass vial, and then about 5.4 g was transferred to the reaction flask through the funnel. The flask was quickly stoppered and removed from the dry box.

While the equipment was swept with N₂ at a moderate rate, the flask was quickly unstoppered and attached to the bottom of the condenser described above. The N₂ flow was then reduced to a slow rate. CFC-113 (about 270 g CCl₂FCClF₂) was added to the addition funnel. The condenser cooling water was started at a medium-high rate, and the magnetic stirrer was turned on at 300 rpm; without turning on the hot plate.

The CCl₂FCClF₂ was added over about 10 minutes with stirring. There was no significant exotherm for this catalyst activation procedure. After addition, the mixture was heated to boil under reflux at 48-52°C with continued stirring. The mixture was stirred at gentle boil for 4 hours, and then allowed to cool while stirring to 25°C, at which point the stirring was stopped.

### B. Isomerization Procedure

After the catalyst settled in the flask, the clear organic liquid was withdrawn with a hypodermic needle using a vacuum flask. The addition funnel was refilled with another portion of CCl₂FCClF₂. The stirrer was turned on at 300 rpm, and the CCl₂FCClF₂ was added at a moderately fast rate, taking about 10 to 15 minutes. The mixture normally reached the boiling point during the addition. After the addition was complete, the mixture was allowed to cool to 20°C while stirring. The stirring was stopped, and the catalyst allowed to settle until the organic liquid was clear. An aliquot of organic liquid was analyzed by gas chromatography.

### C. Catalyst Life

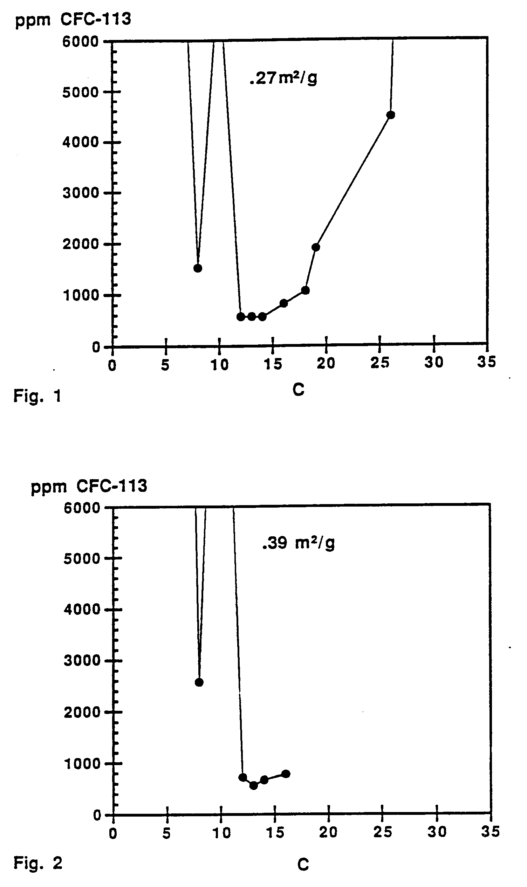

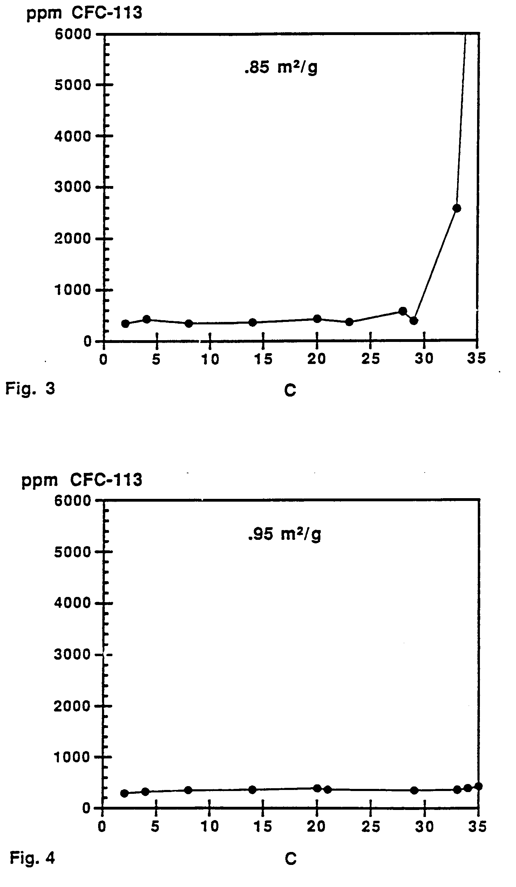

Different samples of non-micropulverized aluminum chloride (surface areas of 0.27 and 0.39 m²/g, respectively), a sample of aluminum chloride having a surface area of 0.85 m²/g, and a sample of micro-pulverized aluminum chloride (surface area of 0.95 m²/gm) were each employed for a series of consecutive isomerisation cycles, and the concentration of CFC-113 (ppm) in the CFC-113a product was monitored. The concentration of CFC-113 in the product is shown as a function of the number of cycles, C, in Figures 1 through 4 for each of the respective samples. The surface area for each AlCl₃ sample was measured using the standard BET nitrogen sorption method described in Example 1 above.

Examples 1 and 2 clearly show that catalysts of this invention are not only highly active (as shown by the rate of isomerization), but also produce high quality product through numerous batch runs.

The examples serve to illustrate particular embodiments of the invention. Other embodiments will become apparent to those skilled in the art from a consideration of the specification or practice of the invention disclosed herein. It is further understood that the invention is not confined to the particular formulations and examples herein illustrated, but it embraces such modified form thereof as come within the scope of the following claims.

## Claims

1. A process for producing compounds of the formula CCl₃CF₃ comprising:
(1) activating a micropulverized anhydrous aluminum trichloride which has a surface area of greater than 0.8 m²/g by treating it with at least 10 g per g of aluminum trichloride of CCl₂FCClF₂ with agitation; and
(2) isomerizing an additional amount of CCl₂FCClF₂ by (i) mixing said additional amount of CCl₂FCClF₂ with said activated catalyst with agitation to provide an initial degree of isomerization, and (ii) cooling the mixture of chlorofluorocarbons and catalyst to a temperature of about 20°C with agitation, to provide a product containing CCl₃CF₃ and less than about 400 ppm of CCl₂FCClF₂.

2. The process of Claim 1 wherein the reactor used for activation is glass or carbon steel; and wherein the reactor used for isomerization is glass or carbon steel.

3. The process of Claim 1 or Claim 2 wherein the isomerization catalyst is activated in the same reactor used for isomerization.

4. The process of Claim 3 wherein the reactor is a glass reactor.

5. The process of Claim 3 wherein the reactor is a carbon steel reactor.

6. The process of any one of Claims 1 to 5 wherein the isomerization is initially conducted at a temperature of at least 47°C and the isomerization rate prior to cooling is at least 10 kg of CCl₂FCClF₂ per kg of AlCl₃ per hour.

7. The process of any one of Claims 1 to 6 wherein at least 500 kg of CCl₂FCClF₂ is isomerized per kg of AlCl₃.

8. The process of any one of Claims 1 to 7 wherein the catalyst is used during isomerization of CCl₂FCClF₂ in a weight ratio of up to 50 g CCl₂FCClF₂ per g AlCl₃, and wherein the catalyst is used for at least 30 isomerization cycles.

9. The process of any one of Claims 1 to 8 wherein the AlCl₃ is sufficiently micropulverized to provide a first order rate constant, after activation, of at least 0.10 minutes⁻¹ at boiling under reflux conditions.

10. The process of any one of Claims 1 to 9 wherein at least 1000 kg CCl₂FCClF₂ is isomerized per kg of AlCl₃.

## Patentansprüche

1. Verfahren zur Herstellung von Verbindungen der Formel CCl₃CF₃, umfassend:
(1) das Aktivieren eines mikropulverisierten, wasser-freien Aluminiumtrichlorids, das eine spezifische Oberfläche von mehr als 0,8 m²/g aufweist, durch Behandlung desselben mit wenigstens 10 g CCl₂FCClF₂ pro g Aluminiumtrichlorid unter Rühren, und
(2) das Isomerisieren einer zusätzlichen Menge von CCl₂FCClF₂ durch (i) Vermischen der zusätzlichen Menge von CCl₂FCClF₂ mit dem aktivierten Katalysator unter Rühren, um einen anfänglichen Isomerisierungsgrad bereitzustellen, und (ii) Abkühlen der Mischung von Chlorfluorkohlenstoffen und Katalysator auf eine Temperatur von etwa 20 °C unter Rühren, um ein Produkt bereitzustellen, das CCl₃CF₃ und weniger als etwa 400 ppm CCl₂FCClF₂ enthält.

2. Verfahren gemäß Anspruch 1, worin der für die Aktivierung verwendete Reaktor aus Glas oder Kohlenstoffstahl besteht, und worin der für die Isomerisierung verwendete Reaktor aus Glas oder Kohlenstoffstahl besteht.

3. Verfahren gemäß Anspruch 1 oder 2, worin der Isomerisierungskatalysator in dem gleichen Reaktor aktiviert wird, der für die Isomerisierung verwendet wird.

4. Verfahren gemäß Anspruch 3, worin der Reaktor ein Glas-Reaktor ist.

5. Verfahren gemäß Anspruch 3, worin der Reaktor ein Kohlenstoffstahl-Reaktor ist.

6. Verfahren gemäß irgendeinem der Ansprüche 1 bis 5, worin die Isomerisierung anfänglich bei einer Temperatur von wenigstens 47 °C durchgeführt wird, und die Isomerisierungsrate vor dem Abkühlen wenigstens 10 kg CCl₂FCClF₂ pro kg AlCl₃ pro Stunde beträgt.

7. Verfahren gemäß irgendeinem der Ansprüche 1 bis 6, worin wenigstens 500 kg CCl₂FCClF₂ pro kg AlCl₃ isomerisiert werden.

8. Verfahren gemäß irgendeinem der Ansprüche 1 bis 7, worin der Katalysator während der Isomerisierung von CCl₂FCClF₂ in einem Gewichtsverhältnis von bis zu 50 g CCl₂FCClF₂ pro g AlCl₃ verwendet wird, und der Katalysator wenigstens in 30 Isomerisierungszyklen verwendet wird.

9. Verfahren gemäß irgendeinem der Ansprüche 1 bis 8, worin das AlCl₃ ausreichend mikropulverisiert ist, um beim Sieden unter Rückfluß-Bedingungen nach der Aktivierung eine Geschwindigkeitskonstante erster Ordnung von wenigstens 0,10 min⁻¹ zu ergeben.

10. Verfahren gemäß irgendeinem der Ansprüche 1 bis 9, worin wenigstens 1000 kg CCl₂FCClF₂ pro kg AlCl₃ isomerisiert werden.

## Revendications

1. Procédé de préparation de composés de formule CCl₃CF₃ consistant a :
(1) activer du trichlorure d'aluminium anhydre micropulvérisé qui a une surface spécifique supérieure à 0,8 m²/g en le traitant avec au moins 10 g de CCl₂FCClF₂ par g de trichlorure d'aluminium sous agitation ; et
(2) isomériser une quantité supplémentaire de CCl₂FCClF₂ (i) en mélangeant ladite quantité supplémentaire de CCl₂FCClF₂ avec ledit catalyseur activé, sous agitation, pour fournir un degré initial d'isomérisation, et (ii) en refroidissant le mélange de chlorofluorocarbones et de catalyseur à une température d'environ 20°C sous agitation, pour fournir un produit contenant CCl₃CF₃ et moins d'environ 400 ppm de CCl₂FCClF₂.

2. Procédé selon la revendication 1, dans lequel le réacteur utilisé pour l'activation est en verre ou en acier au carbone ; et dans lequel le réacteur utilisé pour l'isomérisation est en verre ou en acier au carbone.

3. Procédé selon la revendication 1 ou la revendication 2, dans lequel le catalyseur d'isomérisation est activé dans le même réacteur que celui utilisé pour l'isomérisation.

4. Procédé selon la revendication 3, dans lequel le réacteur est un réacteur en verre.

5. Procédé selon la revendication 3, dans lequel le réacteur est un réacteur en acier au carbone.

6. Procédé selon l'une quelconque des revendications 1 à 5, dans lequel l'isomérisation est initialement effectuée à une température d'au moins 47°C et la vitesse d'isomérisation avant le refroidissement est d'au moins 10 kg de CCl₂FCClF₂ par kg de AlCl₃ par heure.

7. Procédé selon l'une quelconque des revendications 1 à 6, dans lequel au moins 500 kg de CCl₂FCClF₂ sont isomérisés par kg de AlCl₃.

8. Procédé selon l'une quelconque des revendications 1 à 7, dans lequel le catalyseur est utilisé pendant l'isomérisation de CCl₂FCClF₂ dans un rapport pondéral de jusqu'à 50 g de CCl₂FCClF₂ par g de AlCl₃, et dans lequel le catalyseur est utilisé pendant au moins 30 cycles d'isomérisation.

9. Procédé selon l'une quelconque des revendications 1 à 8, dans lequel le AlCl₃ est suffisamment micropulvérisé pour fournir une constante de vitesse de premier ordre, après activation, d'au moins 0,10 minute⁻¹ à l'ébullition dans des conditions de reflux.

10. Procédé selon l'une quelconque des revendications 1 à 9, dans lequel au moins 1000 kg de CCl₂FCClF₂ sont isomérisés par kg de AlCl₃.
